# EUROPEAN PATENT SPECIFICATION

(11) **EP 0 893 956 B1**
(45) Date of publication and mention of the grant of the patent: **27.08.2003**
(21) Application number: 96940731.1
(22) Date of filing: 11.12.1996
(51) Int. Cl.: A41B 9/02

(54) **MEN'S BODY TEMPERATURE CONTROLLING PANTS**
HERRENHOSE MIT STEUERUNG DER TEMPERATUR DES KÖRPERS
CALE ON PERMETTANT D'AGIR SUR LA TEMPERATURE DU CORPS CHEZ UNE PERSONNE DU SEXE MASCULIN

(30) Priority: 12.12.1995 KR 9541021 U
(43) Date of publication of application: 03.02.1999
(73) Proprietor: CHUNG, Seun Yung, Kwangmyung City, Kyeongki-Do 423-060 (KR)
(72) Inventor: CHUNG, Seun Yung, Kwangmyung City, Kyeongki-Do 423-060 (KR)
(74) Representative: Coulson, Antony John
(86) International application number: PCT/KR96/00238
(87) International publication number: WO 97/021363

(56) References cited:
- DE-A- 4 318 302
- US-A- 4 345 337

## Description

This invention relates to men's pants.

The invention makes it possible to promote the production of men's testosterone by maintaining proper body temperature and testicular temperature and also makes it possible to prevent various skin diseases by separation of the body, testicles, penis and hips. These matters were also addressed in previous inventions of the present inventor, including that described in US Patent No. 5,283,912.

In US 5,283,912, men's pants have a small hole to separate the testicles from the body, a fixed penis ring to separate the penis from the testicles, and a double waist belt to make it possible to urinate by lowering a front belt attached to a rubbing cloth. That invention contributed somewhat to the user's health and hygiene by promoting airiness around the genitals.

The prior invention, however, gave rise to some difficulties in separating the testicles from the body with the small hole, which gave an aversion to users. Further, the double belt caused an oppressive feeling to the stomach, airiness was not so great due to the double structure of the outer cloth and the rubbing cloth, except for the hole part, and the production cost was high on account of the double waist belt.

Also, the penis ring of previous inventions was inconvenient to use and detrimental to the user's safety because of its fixed location regardless of the different sizes and locations of individual penises. The round ring was deficient in separation and caused aversion.

Moreover, a significant problem was that the old and the weak found the pants hard to use due to their complicated structure, and the testicles and penis were apt to slip out of the hole and ring respectively because of lack of caution while using.

The present invention improves the above-mentioned functions and addresses deficiencies by altering the structure of the pants. Complete separation of the testicles from the body, the penis from the testicles, and the joint of both hips is possible in accordance with this invention.

Thus this invention makes it possible to promote a user's health by maintaining an ideal body temperature at any time and any place and by eliminating sweat around the genitals during wearing of the pants.

In accordance with this invention, a Y or U type separation band is attached to the inside of conventional men's pants, so that in use the testicles are separated automatically from the body without any specific effort.

The edge of a Y type separation band may comprise elastic rubber band, and it is flexible with respect to the location of individual testicles. Both sides of the Y shaped band are open to increase airiness, and pads may be attached to both sides to separate the testicles, body and hips if the Y type band sinks to the joint part. The lower part of the Y type separation band functions not only for separation from the hips, but also to massage the prostate gland when walking.

The pants may comprise a penis band comprising a double elastic rubber band sewn to an upper waist band, and with two attached clips designed to adjust the location and size of the penis hole. Thus the shape of the penis band looks like the letter I, and does not cause any aversion for the user. Its lower part below the penis hole increases the separation between the penis and the testicles.

An advantage of the pants of this invention is easy urination by lowering the waist band in a state of separation of the penis and the testicles.

Embodiments of the present invention will now be described with reference to the accompanying drawings, in which:
- FIGURE 1: is a rear view of pants in accordance with a first embodiment of the present invention, with part of the back of the pants cut away;
- FIGURE 2: is a front view of the pants of Figure 1 with part of the front of the pants cut away;
- FIGURE 3: is a front view of pants in accordance with a second embodiment of the present invention, with the cover cut away;
- FIGURE 4: is a front view of pants in accordance with the present invention; and,
- FIGURE 5: is a cross-section through pants in accordance with the present invention in use.

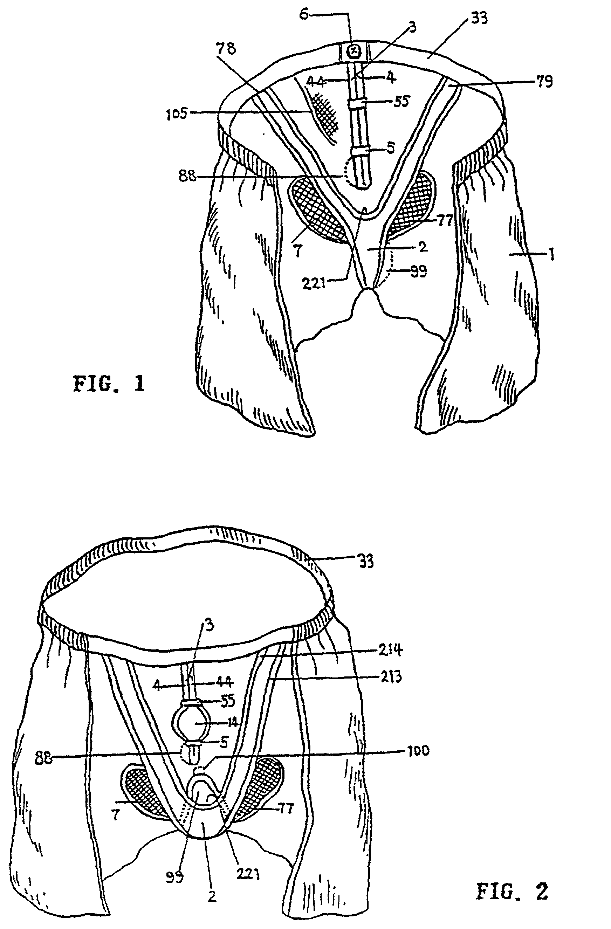

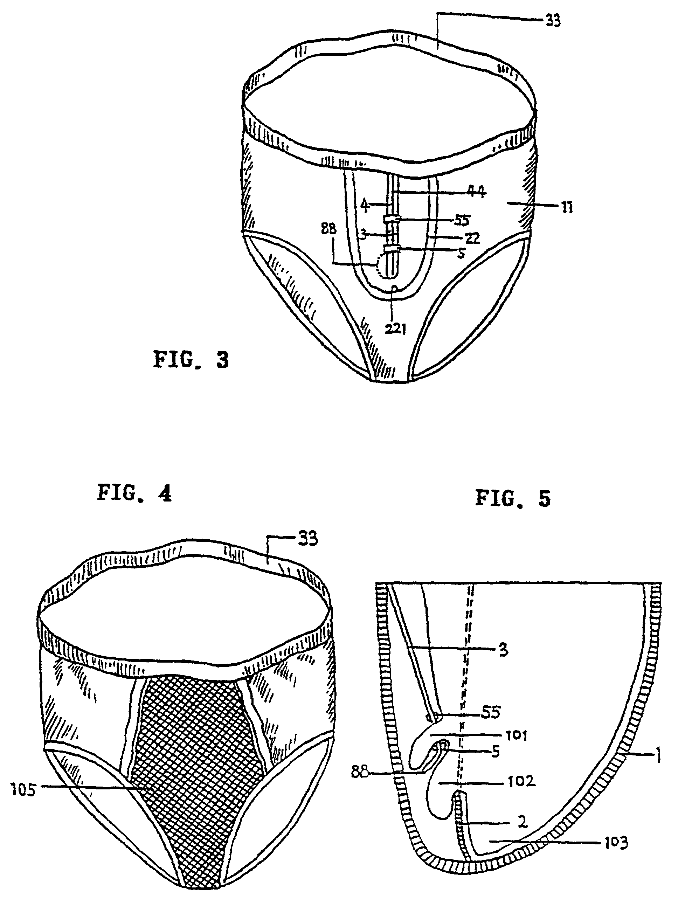

In Figure 1, a Y type separation band 2 with a part 221 in the form of an open inverted arch is attached to a pants main body 1. In Figure 3, a U type separation band 22 with a part 221 in the form of an open inverted arch is attached to a pants main body 11.

In figure 1, the Y type separation band 2 has separation pads 7, 77 attached thereto to promote separation.

The edge of the separation band 2 is a treated elastic rubber band 213, 214. An upper part 78, 79 of the separation band 2 is sewn to the front waist band 33 of the pants. A lower part 99 of the separation band 2 is sewn to a hip location 100 inside the rear part of the pants.

The penis band 3 is made with an elastic rubber band 4,44 in a double I shape. Two clips 5,55 are applied to the penis band 3, which is sewn to a center part 6 of the waist band.

Reference numeral 103 indicates the body of a user. As shown in Figure 5, a penis hole 14 is formed with two clips 5,55 on the penis band 3, with a lower part 88 below the clip 5 increasing the separation between the penis 101 and the testicles 102.

For the user's convenience, the penis band 3 can be attached to the centre part of the waist band 6 with a snap button.

The invention provides men's pants which promote the user's health by maintaining the temperature of testicles below 33°C for greater production of testosterone which controls men's energy and vigour, which prevent skin diseases around the genitals by eliminating sweat, and which increase airiness.

## Claims

1. Men's pants comprising means for separating the user's testicles (102) from the user's body (103) **characterised in that** the said means comprises a Y-shaped separation band (2) attached to the inside of the pants.

2. Men's pants as claimed in claim 1 and further comprising separation pads (7, 77) attached to the separation band (2).

3. Men's pants as claimed in claim 2, wherein the separation pads (7, 77) extend laterally from said Y-shaped separation band adjacent to a position at which the arms of the Y join the upright thereof.

4. Men's pants as claimed in any one of claims 1 to 3, wherein the arms of the Y form an open inverted arch (221).

5. Men's pants as claimed in claim 4, wherein the upright of the Y, which forms a lower part (99) of the separation band, is attached to a hip location inside the rear part of the pants.

6. Men's pants comprising means for separating the user's testicles (102) from the user's body (103) **characterised in that** the said means comprises a U-shaped separation band (22) attached to the inside of the pants.

7. Men's pants as claimed in claim 6, wherein the U-shaped separation band (22) is in the form of an open inverted arch (221).

8. Men's pants as claimed in any preceding claim and further comprising a penis band (3), the penis band comprising a penis hole (14) for retaining the penis (101), the location of the penis hole (14) being defined by the position of first (5) and second (55) clips on the penis band.

9. Men's pants as claimed in claim 8, wherein the location of each of the first (5) and second (55) clips on the penis band is adjustable such that the size and location of the penis hole can be varied.

10. Men's pants as claimed in claim 8 or claim 9, wherein the penis band (3) comprises an elastic rubber band.

11. Men's pants as claimed in any one of claims 8 to 10, wherein the penis band is detachably attached to the centre of the waistband.

12. The use of men's pants as claimed in any preceding claim to maintain the temperature of the testicles (102) of the wearer of the pants below 33°C.

## Patentansprüche

1. Herrenunterhose mit einer Einrichtung zum Trennen der Hoden (102) des Benutzers von dem Körper (103) des Benutzers, **dadurch gekennzeichnet, daß** die Einrichtung ein Y-förmiges Trennband (2) aufweist, das an der Innenseite der Unterhose befestigt ist.

2. Herrenunterhose nach Anspruch 1 mit ferner Trennkissen (7, 77), die an dem Trennband (2) befestigt sind.

3. Herrenunterhose nach Anspruch 2, wobei sich die Trennkissen (7, 77) seitlichen von dem Y-förmigen Trennband weg zu einer Stelle erstrecken, an der die Arme des Y in den senkrechten Teil davon übergehen.

4. Herrenunterhose nach einem der Ansprüche 1 bis 3, wobei die Arme des Y einen offenen invertierten Bogen (221) bilden.

5. Herrenunterhose nach Anspruch 4, wobei der senkrechte Teil des Y, der einen unteren Teil (99) des Trennbandes bildet, an einer Hüftposition innerhalb des hinteren Teils der Unterhose befestigt ist.

6. Herrenunterhose mit einer Einrichtung zum Trennen der Hoden (102) des Benutzers von dem Körper (103) des Benutzers, **dadurch gekennzeichnet, daß** die Einrichtung ein U-förmiges Trennband (22) aufweist, das an der Innenseite der Unterhose befestigt ist.

7. Herrenunterhose nach Anspruch 6, wobei das U-förmige Trennband (22) die Form eines offenen invertierten Bogens (221) hat.

8. Herrenunterhose nach einem der vorausgehenden Ansprüche mit ferner einem Penisband (3), wobei das Penisband (3) eine Penisöffnung (14) zur Aufnahme des Penis (101) aufweist, wobei die Stelle der Penisöffnung (14) durch die Position einer ersten (5) und einer zweiten (55) Klammer an dem Penisband begrenzt wird.

9. Herrenunterhose nach Anspruch 8, wobei die Stelle sowohl der ersten (5) als auch der zweiten (55) Klammer an dem Penisband in der Weise einstellbar ist, daß die Größe und Position der Penisöffnung (14) verändert werden kann.

10. Herrenunterhose nach Anspruch 8 oder 9, wobei das Penisband (3) ein elastisches Gummiband aufweist.

11. Herrenunterhose nach einem der Ansprüche 8 bis 10, wobei das Penisband lösbar an der Mitte des Bundes befestigt ist.

12. Verwendung der Herrenunterhose nach einem der vorausgehenden Ansprüche zur Aufrechterhaltung der Temperatur der Hoden (102) des Trägers der Unterhose unterhalb 33°C.

## Revendications

1. Caleçon pour homme comprenant des moyens pour séparer les testicules de l'utilisateur (102) par rapport au corps de l'utilisateur (103), **caractérisé par le fait que** les dits moyens comprennent une bande de séparation en forme de Y (2) fixée à l'intérieur du caleçon.

2. Caleçon pour homme selon la revendication 1, comprenant en outre des tampons de séparation (7, 77) fixés à la bande de séparation (2).

3. Caleçon pour homme selon la revendication 2, dans lequel les tampons de séparation (7, 77) s'étendent latéralement depuis la bande de séparation en forme de Y jusqu'à proximité d'une position dans laquelle les bras du Y atteignent le dessus de celui-ci.

4. Caleçon pour homme selon l'une quelconque des revendications 1 à 3, dans lequel les bras du Y forment une arche ouverte retournée (221).

5. Caleçon pour homme selon la revendication 4, dans lequel la partie verticale du Y, qui forme une partie basse (99) de la bande de séparation, est fixée à une position de hanche à l'intérieur de la partie arrière du caleçon.

6. Caleçon pour homme comprenant des moyens pour séparer les testicules de l'utilisateur (102) par rapport au corps de l'utilisateur (103), **caractérisé par le fait que** les dits moyens comprennent une banda de séparation en forme de U (22) fixée à l'intérieur du caleçon.

7. Caleçon pour homme selon la revendication 6, dans lequel la bande de séparation en U (22) est sous forme d'une arche ouverte retournée (221).

8. Caleçon pour homme selon l'une quelconque des revendications précédentes, comprenant en outre une bande pour pénis (3), la bande pour pénis comprenant un trou pour le pénis (14) pour maintenir le pénis (101), la position du trou pour le pénis (14) étant définie par la position de premier (5) et second (55) clips sur la bande pour pénis.

9. Caleçon pour homme selon la revendication 8, dans lequel la position de chacun des premier (5) et second (55) clips sur la bande pour pénis est ajustable de façon que l'on puisse modifier la taille et la position du trou pour pénis.

10. Caleçon pour homme selon l'une des revendications 8 et 9, dans lequel la bande pour pénis (3) comprend une bande élastique en caoutchouc.

11. Caleçon pour homme selon l'une quelconque des revendications 8 à 10, dans lequel la bande pour pénis est fixée de façon amovible au centre de la bande de ceinture.

12. Utilisation d'un caleçon pour homme selon l'une quelconque des revendications précédentes pour maintenir la température des testicules (102) du porteur du caleçon en-dessous de 33°C.
